Europäisches Patentamt

European Patent Office

Office européen des brevets

(19)

(11) Veröffentlichungsnummer: **0 205 931**
**B1**

(12) **EUROPÄISCHE PATENTSCHRIFT**

(45) Veröffentlichungstag der Patentschrift: **22.11.90**

(21) Anmeldenummer: **86106919.3**

(22) Anmeldetag: **21.05.86**

(51) Int. Cl.⁵: **A 61 B 5/103,** A 61 B 5/08

(54) Vorrichtung zur Messung der Lebensfunktion eines Menschen, insbesondere eines Säuglings.

(30) Priorität: **23.05.85 DE 3518541**

(43) Veröffentlichungstag der Anmeldung:
**30.12.86 Patentblatt 86/52**

(45) Bekanntmachung des Hinweises auf die
Patenterteilung:
**22.11.90 Patentblatt 90/47**

(84) Benannte Vertragsstaaten:
**AT BE CH DE FR GB IT LI LU NL SE**

(56) Entgegenhaltungen:
**DE-A-3 009 216        US-A-4 438 771
US-A-3 760 794        US-A-4 474 185
US-A-4 365 636**

**MEDICAL & BIOMEDICAL ENGINEERING &
COMPUTING, Band 21, Nr. 6, November 1983,
Seiten 781-782, Stevenage, Herts, GB; P.
BOUCHET et al.: "Un actomètre destiné à la
surveillance du nouveau né"**

**Die Akte enthält technische Angaben, die nach
dem Eingang der Anmeldung eingereicht
wurden und die nicht in dieser Patentschrift
enthalten sind.**

(73) Patentinhaber: **Reents, Heinrich, Prof. Dr. Ing.
Magnolienweg 23
D-4750 Unna (DE)**

(72) Erfinder: **Reents, Heinrich, Prof. Dr. Ing.
Magnolienweg 23
D-4750 Unna (DE)**

(74) Vertreter: **Köchling, Conrad-Joachim et al
Patentanwälte Dipl.-Ing. Conrad Köchling, Dipl.-
Ing. Conrad-Joachim Köchling Fleyer Strasse
135
D-5800 Hagen 1 (DE)**

EP 0 205 931 B1

**Beschreibung**

Die Erfindung betrifft eine Vorrichtung zur Messung der Lebensfunktionen eines Menschen, insbesondere eines Säuglings, mittels einer durch Druckänderungen wirkenden Unterlage als Lebenszustands-Monitor, die auch Temperatur-, Feuchtigkeits- etc.-sensoren aufweist und auf einem mit der durch Druckänderungen wirkenden Unterlage verbundenen Meß- und Anzeigevorrichtung wirkt.

Aus DE—A—30 09 216 sind bereits flächige Sensoren bekannt. Diese basieren jedoch auf dem elektrostatischen Prinzip. Das System arbeitet jedoch nicht gewichtsunabhängig und unabhängig von der Mattengröße. Dies ist jedoch eine wesentliche Anforderung. Während das Gewicht erwachsener Personen um ca. 100% variiert, nimmt das Gewicht eines Babys im Laufe der ersten zwei Jahre um den Faktor 6—18 zu. Bei einer Frühgeburt kann der Faktor noch höher sein. Arbeitet das System nicht gewichtsunabhänig ist eine laufende Nachjustage unumgänglich. Weiterhin arbeitet das System mit einem passiven Sensor. Er muß mit Fremdenergie versorgt werden. Demgegenüber arbeitet das in dieser Erfindung vorgestellte System gewichtsunabhängig und unabhängig von der Mattengröße. Es basiert auf einem aktiven Sensor. Die Fremderregung des aktiven Sensors erfolgt durch die Bewegungsenergie des Muskelapparates. Durch den symmetrischen Aufbau der Sensormatte ist die Empfindlichkeit des Gesamtsystems auf beiden Seiten der Sensormatte gleich groß. Die Unabhängigkeit von der Mattengröße ist bei einem Säugling eine wesentliche Anforderung, bedingt durch das außerordentliche Wachstum während der ersten Lebensmonate.

Eine Detektionsschaltung bestehend aus Vorverstärker, Differentialverstärker, diverser Filter sowie Komparatoren wurde auch in der Patentschrift US—A—4 438 771 dargestellt. Das vorgestellte System arbeitet jedoch nicht gewichtsunabhängig und nicht als aktiver Sensor. Es beinhaltet keine doppelte Differenzeingangsstufe.

Die Patentschrift US—A—4 356 636 beschreibt ein Verfahren der Bildmusteranalyse, mit deren Hilfe Atemstillstände erkannt werden können. Es wird eine Analyse der Flankensteilheit durchgeführt. Die Analyse der Flankensteilheit reicht jedoch nicht aus als Maßstab eines Bildmusters. Aufgrund dessen wurde in der vorgestellten Erfindung eine Rechnerschnittstelle im System eingebaut, die die Analyse der Ausgangssignale nach unterschiedlichsten Kriterien erlaubt. So ist vorgesehen, die Bildmustererkennung anhand vorgewählter Toleranzkurven des Normalzustandes, die im Speicher abgelegt sind, durchzuführen. Neben der Frequenzanalyse, der Analyse der Amplitude und Flankensteilheit ist der Einsatz der Gaußschen Fehlerquadratmethode beim Vergleich der Bildmuster ein brauchbares Instrumentarium, verbunden mit einer Tendenzanalyse.

Alle oben genannten Erfindungen beinhalten lediglich die Überwachung der Atemfrequenz und der Pulsfrequenz. Weitere relevante Meßgrößen wie Temperatur oder Feuchtigkeit werden nicht erfaßt, obwohl gerade diese Größen Hinweise auf Störungen des Säglings ergeben können. So geht in vielen Fällen der atypischen Apnoe ein plötzlicher Temperaturanstieg voraus. Darüberhinaus ist keine Aufteilung in einzelne Felder zur Erkennung der Lage des Menschen vorgesehen. Diese Segmentierung der Sensormatte ist jedoch eine wesentliche Voraussetzung zur Unterscheidung zwischen Atembewegungen, Herzbewegungen und anderen Muskelkontraktionen. Diese Unterscheidung ist wesentlich zum Erkennen atypischer Apnoen, die durch ein Zittern im Bereich der Atem- und Herzfrequenz sowie deren Amplituden gekennzeichnet sind.

Die wesentlichen Unterschiede zum Stand der Technik liegen somit in der symmetrischen Ausführung der Sensormatte und der doppelten Differenzeingangsstufe, wodurch die Funktion der Sensormatte weitestgehend unabhängig von der Gewichtsbelastung des Patienten und unabhängig von der Größe der Matte erfüllt wird.

Darüberhinaus ist der Sensor in Form eines aktiven Senors ausgeführt. Er benötigt keine Hilfsenergie. Dieser Eigenschaft ist unter dem Gesichtspunkt Sicherheit eine hohe Bedeutung zuzumessen. Darüberhinaus gestattet die Rechnerschnittstelle den Einsatz künstlicher Intelligenz, so daß weitere Kriterien als Flankensteilheit, Frequenz, Amplitude zu eindeutigen Identifizierung der Atem- und Herzsignale herangezogen werden können. Weiterhin fehlen bei den aus dem Stand des Technik bekannten Geräten integrierte Temperatur- und Feuchtigkeitssensoren. Wichtige Störungen, die durch eine plötzlich erhöhte Temperatur sowie das Versagen der Körperverschlußöffnungen angezeigt werden, können damit nicht erfaßt werden.

Aufgabe der Erfindung ist es, ein unversell anzeigendes Lebensfunktionsmeßgerät anzugeben, das unter allen Umständen und unter Einsatz der modernsten elektronischen Verstärkungstechnik Auskunft über den Normalzustand oder das Abweichen vom Normalzustand, insbesondere über ein Abweichen in absehbarer Zeit gibt.

Die Aufgabe wird dadurch gelöst, daß die durch Druckänderungen wirkende Unterlage ein Signal abgibt, das die Brustkorb- bzw. Zwerchfellbewegung beim Atmen und die Bewegung der Herzmuskeln wiedergibt sowie insbesondere mittels eines Hilfsensors ein Signal für den Pulsschlag liefert. Hierdurch sind die drei wesentlichen Anzeigen für eine normale Lebensfunktion wiedergegeben und geben den ersten groben Raster für die Anzeige, ob ein Lebewesen schwere Störungen der Lebensfunktionen aufweist oder nicht, bei der Anzeige, daß schwere Störungen vorliegen, kann ein Mensch oder auch für die ferne Zukunft vorausgedacht, eine entsprechende, medizin-technisch wirkende Einrichtung in Funktion treten und alle Wiederbelebensaktionen vornehmen, die notwendig sind.

In Ausgestaltung der Erfindung ist vorgesehen, daß die Signale der durch Druckänderungen wirkenden Unterlage einer Bildmustererkennung einer anhand vorgewählter Toleranzkurven des Normalzustandes im Speicher enthaltenen Einheit unterzogen werden. Hierdurch ist es möglich, Abweichungen von dem normalen Lebensrythmus eines Lebenswesens abweichende Anzeichen aufzunehmen und zu erkennen. Als Bildmuster wird ein Normallebewesen in den entsprechenden Aparator, d.h. einen entsprechenden Computer eingespeist und alle außerhalb des Toleranzbereichs liegenden Abweichungen angezeigt. Dabei werden vorzugsweise zwei Teoleranzbereiche normale, noch im Rahmen des Erträglichen liegenden Abweichungen und eine tödliche Störung anzeigende Abweichungen unabhängig voneinander angezeigt. Damit kann die persönliche Konstitution des Lebewesens, das überwacht wird, berücksichtigt werden.

In weiterer Ausgestaltung der Erfindung ist vorgesehen, daß die Signale der durch Druckänderungen wirkenden Unterlage einer Frequenzanalyse unterzogen werden, des weiteren auch einer Analyse auf die Amplitudenhöhe unterzogen werden. Durch diese beiden Kriterien ist es möglich, die Bewegung der einzelnen Muskeln, z.B. der Herzmuskeln, der großen Muskeln, z.B. der Bein- und Armmuskeln, der Muskeln der Atmung und der übrigen Muskeln zu differenzieren. Eine Anzeige eines ungewöhnlichen Verhaltens erfolgt erste bei Überschreiten der entsprechenden Toleranzgrenzen. Es wird vorteilhafterweise berücksichtigt, daß die Frequenzen, z.B. des Herzmuskels und der Atmungsmuskeln in der Regel voneinander abweichen. Sie können unter besonderen Umständen gleich sein, dies ist aber in der Regel ein Anzeichen einer gestörten Atmungsaktivität, insbesondere eines Sauerstoffmangels.

In weiterer Ausgestaltung der Erfindung ist vorgesehen, daß die Signalauswertung mittels einer Differenzschaltung erfolgt. Die Differenzschaltung erlaubt in vorteilhafter Weise unterschiedliche Gewichte des Lebewesens, dessen Lebensfunktionen überwacht und kontrolliert werden, von seinem Gewicht unabhängig anzuzeigen. So ergibt sich eine universelle Einsatzmöglichkeit ohne, daß auf das jeweilige Lebewesen abgestimmt, eingestellt werden muß. Die Forderung, daß durch ein einfaches Einstecken eines Steckers ohne weitere Feinabstimmung eine Überwachung möglich ist, einer einfachen Bedienungsweise wird hiermit in bester Weise Rechnung getragen.

In weiterer Ausgestaltung der Erfindung ist vorgesehen, daß die durch Druckänderungen wirkende Unterlage gewichtsunabhängig und von der Größe der Matte unabhängig als aktiver Sensor wirkt, dessen von dem menschlichen Körper abgegebene Bewegungsenergie verstärkt und der Bildmusteranalyse der Frequenzanalyse oder der Amplitudenanalyse unterzogen wird. Dies ist die vorteilhaft mögliche Ausführung der erfindungsgemäßen Lebensfunktionsüberwachungsvorrichtung. Eine weitere Ausbildung kann darin bestehen, daß die gewichtsunabhängig arbeitende Matte in verschiedene Felder aufgeteilt ist, so daß es gleichzeitig möglich ist, die Lage des Menschen, dessen Lebensfunktionen überwacht werden, festzustellen. Insbesondere für ältere Menschen ist es hierdurch möglich, einen Zustand voranzuzeigen, der ein Herausfallen aus dem Krankenbett voranzeigt.

In weiterer Ausgestaltung der Erfindung ist vorgesehen, daß die analysierten Einzelsignale gesondert angezeigt werden und einzeln Alarme auslösen. So ist es möglich, die vielen unterschiedlichen Gefahrenzustände eines Lebewesens, insbesondere eines bettlägrigen Patienten oder eines Kleinkindes so anzuzeigen, daß das Pflegepersonal vor dem Ereigniseintritt reagieren kann. Dabei ist es besonders vorteilhaft, daß die Einzeilsignale einer Tendenzanalyse unterzogen werden und daß das Ergebnis der Tendenzanalyse anzeigt und der Einzelalarm-Anzeigevorrichtung aufgegeben wird. Dies erweitert die Möglichkeiten der Anzeigen der Lageänderung und der Möglichkeit einer Verschlechterung der Situation erheblich. Die Tendenzanalyse ist ein für Industrieanlagen, insbesondere für thermische Anlagen, ein bereits bekanntes System, das unbedenklich angewendet werden kann und in bisher unerreichter Weise auch für Menschen die Annäherung an bedenkliche Zustände anzeigt.

Die Einzelsignale werden durch Fernübertragung auf eine stationäre oder mobile Überwachungsstation übertragen und hintereinander oder gleichzeitig angezeigt und können dort Einzelalarme auslösen. Dies ist die dem hautigen Trend der Personaleinsparung entgegenkommende Möglichkeit einer Überwachung durch ein Minimum an Personal, ohne, daß eine Beeinträchtigung des überwachenden Patienten stattfindet.

Zur Durchführung des Verfahrens ist eine Vorrichtung vorgesehen, die die Lebensfunktion eines Menschen, insbesondere eines Säuglings mittels einer kapazitiv arbeitenden Unterlage als Lebenszustands-Monitor mit einer mit der Unterlage verbundenen Meß- und Anzeigevorrichtung zur Aufbereitung und Verstärkung des von der kapazitiv arbeitenden Unterlage übertragenen Signale aufweist. Die Vorrichtung beinhaltet einen Frequenzanalysator für einfache Aufgabenstellungen, einen Amplitudenanalysator, wenn eine weitergehende Sicherheit der Anzeige erforderlich ist und einen Bildmaster-Analysator, der einen Vergleich zu einer Normalreaktion eines Lebewesens, insbesondere eines Säuglings, beinhaltet und im Rahmen vorgegebener Toleranzgrenzen vergleicht sowie entsprechende Verstärkungsstrecken für die Einzelsignale. Diese sind vorzugsweise gleich aufgebaut, um die Kosten der gesamten Vorrichtung entsprechend zu senken.

Die Verstärkungsstrecken weisen eine Spannungsquelle auf, die gegenüber der durch Druckänderungen wirkenden kapazitiven Unterlage eine Trennstelle aufweist. So ist gewährleistet, daß eine Fehlschaltung keinen tödlichen Stromstoß für das Lebewesen auslösen kann. Dies hat sich in der Vergangenheit in der Medizintechnik als immer wieder gefährlich, wenngleich angeblich ausgeschlossen, herausgestellt. Die Trennstelle ist daher, ebenso wie die Ausbildung der Sensormatte, als aktiv wirkendendes Element ganz wesentlich zur Beurteilung der gesamten Erfindung.

Die Schaltungstechnik der einzelnen Verstärkungsstrecken wird anschließend beschrieben und ist in allen Einzelheiten aus dem Patentanspruch und der Beschreibung zu entnehmen.

In weiterer, die Schaltungstechnik der einzelnen Verstärkungstechnik übersteigende Ausbildung ist vorgesehen, daß der Pulsschlag des Kindes unabhängig von sonstigen Sensoren durch einen auf der Haut aufgebrachten Sensor drahtlos zu einem Empfänger, insbesondere der kapazitiv wirkenden, die Lebensfunktion aufnehmenden, Matte übertragen wird. So ist eine noch weitere genaue Differenzierung der Lebensfunktionssignale möglich.

In weiterer Ausgestaltung der Erfindung ist vorgesehen, daß die einzelnen Lebensfunktionssignale auf eine zentrale Anzeigeneinheit übergehen, die von einer Aufsichtsperson überwacht werden kann. So ist einer bisher nur Intensivstationen mögliche Überwachung der einzelnen Patienten gegeben, ungleich preiswerter und auch gleichzeitig ohne Patientengefährdung durch Kabel, Schläuche etc. möglich ist. Die tatsächliche Ausführung ist einfach durch Systeme, wie sie beispielsweise in der Luft- und Seefahrt zur Auffindung und Bergung von Personen bereits eingeführt sind, möglich. Diese Systeme arbeiten weitestgehend störungsfrei und durch großserienmäßig hergestellte preiswerte Geräte.

Die Erfindung wird in Zeichnungen in einer bevorzugten Ausführungsform gezeigt, wobei aus den Zeichnungen weitere vorteilhafte Einzelheiten der Erfindung entnehmbar sind.

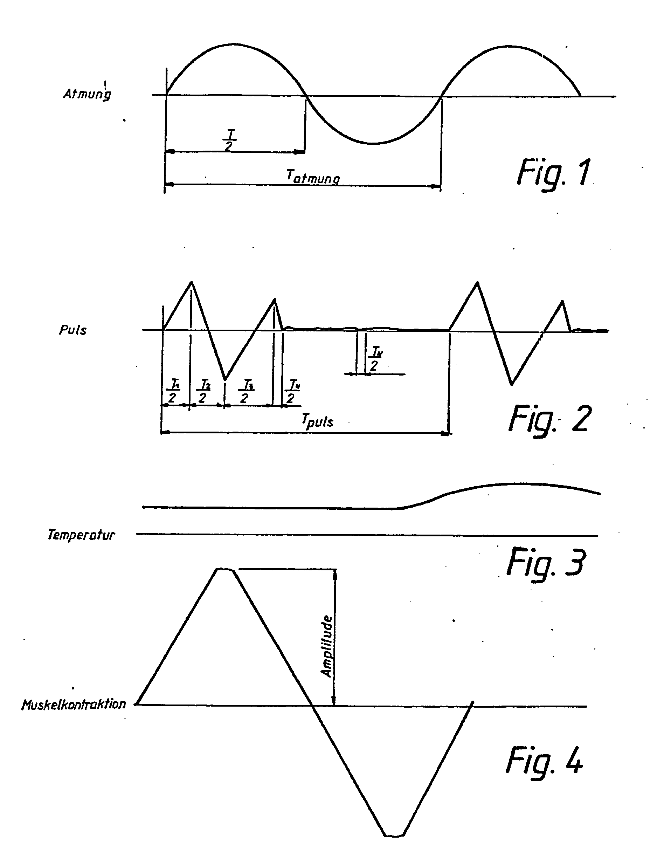

Die Fig. 1—4 zeigen idealisierte Signalverläufe der Meßgrößen.

In Fig. 1 ist der idealisierte Verlauf eines typischen Atmungssignals dargestellt, wobei die Zeitdauer eines Atemzuges als $T_{atmung}$ eingezeichnet ist. Die halbe Periodendauer eines Atemzuges ist bei der als Sinusschwingung idealisierten Darstellung als $T/2$ gekennzeichnet.

Der in Fig. 2 dargestellte idealisierte Verlauf des Pulses gleicht eher einer abklingenden dreieckförmigen Schwingung mit Pausen. Die Wiederholzeit des Impulses ist mit $T_{puls}$ eingetragen. Die einzelnen kennzeichnenden Anstiegs- und Abstiegsflanken des kennzeichnenden Signalteils sind mit $T1/2$, $T2/2$, $T3/2$, $T4/2$ und $T_{n/2}$ gekennzeichnet. Diese gekennzeichneten Signalteile können auch als Teile von überlagerten Schwingungen gedeutet werden, woraus dann erkennbar ist, daß der Puls einen wesentlich höheren Anteil an höherfrequenten Schwingungen aufweist als das Signal der Atmung, obwohl die Wiederholzeit der Atmung und die des Pulses durchaus in der gleichen Größenordnung liegen kann.

Fig. 3 dagegen zeigt einen angenommenen Signalverlauf der Temperatur. Erkennbar ist, daß sich der Signalverlauf der Temperatur sich nicht unbedingt periodisch ändern muß und auch die Änderung des Signals in einer wesentlich kleineren Größenordnung als die Amplituden des Pulssignals oder Atmungssignals liegen.

Dagegen weist der in Fig. 4 dargestellte Signalverlauf der Muskelkontraktion eine wesentlich höhere Amplitude als die übrigen Signale auf. Dieses auffällige Merkmal ist durch die eingezeichnete Amplitudenhöhe kenntlich gemacht. Aus dem Verlauf des Signals läßt sich auf einen Frequenzinhalt schließen, der sich ebenfalls von den übrigen Signalen unterscheidet. Erfindungsgemäß wird aus dem Meßsignal durch die Analyse der unterschiedlichen Frequenzen und Amplituden die Information gewonnen zur Überwachung der verschiedenen Lebensfunktionen.

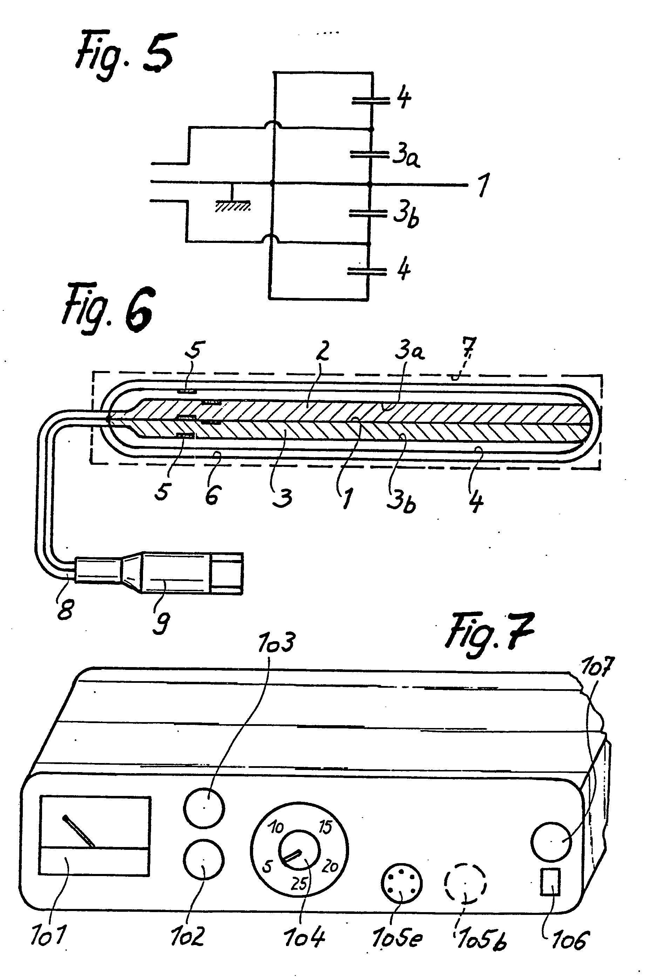

Das Ersatzschaltbild eines geeigneten Meßsensors als Signalgeber ist in Fig. 5 dargestellt.

Das elektrische Feld baut sich zwischen der mittleren Alufolie 1 und den beiden äußeren Alufolien 3a und 3b einerseits sowie zwischen der Abschirmung 4 und den beiden äußeren Alufolien 3a und 3b andererseits auf. Durch die Parallelschaltung von vier Kondensatoren wird eine Erhöhung der Empfindlichkeit erreicht. Dies ist in Figur 5 schematisch gezeigt. Die Abschirmfolie ist innen isolierend und außen leitend. Sie ist ebenso wie die mittlere Folie 1 auf Massepotential gelegt. Die Abschirmfolie 4 erfüllt damit folgende Funktionen:

es kann kein elektrisches Feld von innen nach außen und von außen nach innen dringen;

sie gibt der Matratze ein gutes, homogenes Aussehen;

durch die Erdung der Matte stellt sie die zweite Schutzstufe für das Kind gegenüber potentiellen Überspannungen dar;

sie erfüllt Kondensatorfunktionen gegenüber den Kondensatorplatten 3a und 3b;

sie dient der Rückstrahlung der Körperwärme, was einen zusätzlichen Nutzen bei Neugeborenen darstellt, die leicht an Unterkühlung leiden.

Die Kondensatorplatten 3a und 3b sind in Form einer beidseitig leitenden Folie aufgebaut. Alle Folien (z.B. Aluminiumfolien) sind gegen Verschieben durch doppelseitige Klebestreifen gesichert. Die Klebestreifen sind nur punktuell angebracht, um die Empfindlichkeit der Matte insgesamt nicht herabzusetzen. Der Abstand zwischen den Kondensatorplatten (Alufolie) 3a und 3b wird durch ein abstandhaltendes Medium hergestellt. Die Verbindung zum Gerät erfolgt über ein abgeschirmtes Koaxialkabel und Diodendenstecker.

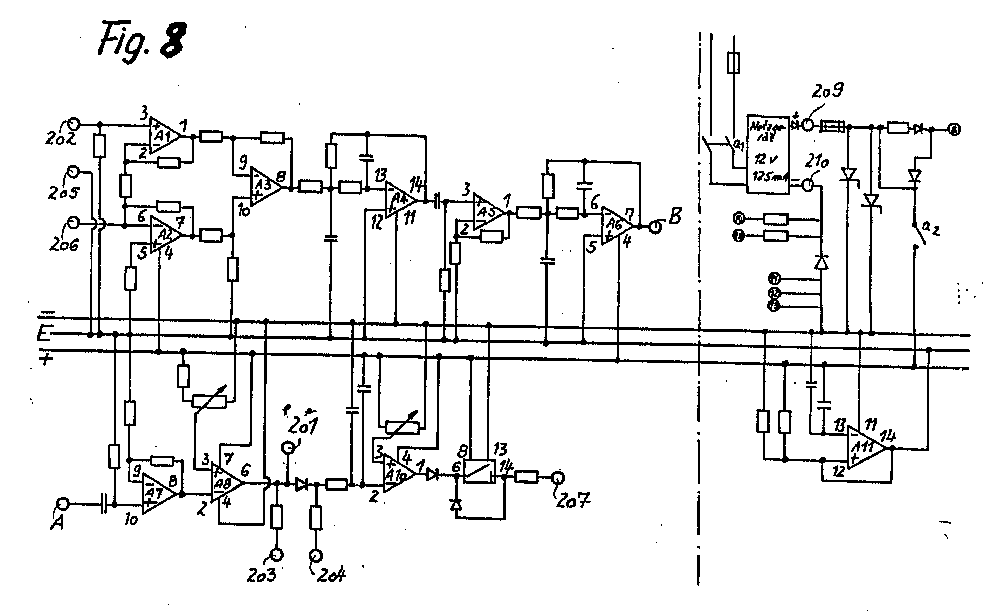

Der Aufbau der Matratze ist in Figur 6 dargestellt.

Mit 1 ist die mittlere Alumiumfolie bezeichnet, die beidseitig leitend ausgebildet ist. Mit 2 ist ein abstandhaltendes Medium bezeichnet. Mit 3a und 3b sind Aluminiumfolien bezeichnet, die beidseitig leitend ausgebildet sind. Mit 4 ist eine weitere Aluminiumfolie bezeichnet, die außen leitend und innen isolierend ausgebildet ist. Mit 5 sind doppelseitig klebende Streifen bezeichnet, die nur punktuell vorgesehen sind. Mit 6 ist eine PVC-Folienhaut bezeichnet, die der Hygiene und Isolierung dient.

Mit 7 ist ein Textilbezug angedeutet, der nicht funktionsbestimmend ist. Mit 8 ist ein abgeschirmtes Koaxialkabel bezeichnet, welches endseitig mit einem fünfpoligen Diodenstecker 9 versehen ist.

In Figur 7 ist schematisch das Gehäuse für das elektronische Überwachungsgerät dargestellt. Das Gehäuse kann aus Aluminium oder auch aus Kunststoff gefertigt sein. Prinzipiell erfüllt das Gehäuse Abschirmfunktionen. Im Gehäuse ist ein Anzeigegerät 101 für den Atmungsimpuls angeordnet.

Des weiteren ist eine grüne Leuchtdiode 102 in der Frontplatte des Gehäuses angeordnet, die synchron zum Atmungsimpuls aufleuchtet. Eine weitere rote Leuchtdiode 103 stellt die optische Alarmanzeige dar. Mit 104 ist ein Zeitintervallgeber (eintellbar) bezeichnet. Mit 105a und 105b sind Diodeneingangsbuchsen zum Anschluß der Sensormatte bezeichnet. Der Ein-Aus-Schalter des Gerätes ist mit 106 bezeichnet, während eine grüne Leuchtdiodenanzeige 107 die Stromversorgung über das Netz anzeigt. Bei Batteriebestrieb bzw. Notbetrieb werden die Leuchtdioden 102 und 107 automatisch ausgeschaltet. In der Gehäuserückseite ist der Netzausgang sowie ein 12 Volt-Batterieeingangsstecker vorgesehen. Die prinzipielle Schaltung der Vorrichtung ist in Figur 8 gezeigt.

Diese Schaltung weist im wesentlichen vier Komponenten auf, nämlich eine Verstärkereinheit, eine Auswerte- und Alarmeinheit, ein Netzteil sowie Notstromversorgung und Batteriebetrieb und einen Geräuschübertragungsmelder und -Empfangseinheit.

Die mittlere Mattenfolie 1 und die äußere Schutzfolie 4 sind über einen Anschlußkontakt 205 der Kontaktleiste mit der Schaltung verbunden und an die künstliche Masse/Erde E angelegt. Die obere Kondensatorfolie und die untere Kondensatorfolie werden jeweils über die Steckerpole 202 bzw. 206 mit der Schaltung verbunden. Die beiden Eingangsverstärker A1 und A2 sind hochohmig ausgelegt. Das Signal wird anschließend einem Differenzverstärker A3 zugeführt. Es wird dort um den Faktor 10 verstärkt. Eine weitere Verstärkung auf dieser Stufe ist nicht sinnvoll, da die angelegten Offsetspannungen den Ausgang von A3 in die Begrenzung führen könnten. Aus dem Signal von A3 werden über eine Tiefpaß der Netzbrumm und die hohen Frequenzen herausgefiltert (A4). Die obere Grenzfrequenz des Tiefpasses liegt ein wenig unter 50 Hz. Der anschließende passive Hochpaß befreit das Signal von Gleichspannungsanteilen, die den vorhergehenden Tiefpaß noch passieren konnten. Die Grenzfrequenz beträgt ca. 1 Hz. In A5 wird das Signal um den Faktor 1000 verstärkt. Durch die hohe Eingangsimpedanz von A5 wird der Hochpaß nicht stark belastet. Durch die Verstärkerstufe A5 kommen wieder Rausch- und Brummanteile in das Signal. Diese werden durch den Tiefpaß A6 wieder herausgefiltert, entsprechend A4. Durch den anschließenden passiven Hochpaß werden die Gleichspannungsanteile wieder herausgezogen, bevor das Signal mit Hilfe von A7 nochmals um den Faktor 200 verstärkt wird. Das Ausgangssignal B ist für eine evtl. vorgesehene Rechnerschnittstelle brauchbar.

Das Signal der Atmung ist nun sowie et verstärkt, daß es über einen Komparator mit einstellbaren Referenzwerten, z.B. über ein Potentiometer, ausgewertet werden kann. Dabei beträgt die Differenz zwischen der Ruhespannung der Matratze (Sensor) und der Referenzspannung 0,02 Volt. Da der Komparator bei einer Spannungsdifferenz von 105 µV noch einwandfrei schaltet, kann die Schaltung noch um einen Faktor 100 empfindlicher gemacht werden. Eine zu hohe Empfindlichkeit führt jedoch zu Eigenschwingungen, weswegen der Arbeitspunkt mit Hilfe eines Oszillokopes exakt eingestellt werden sollte. Während bei den Bausteinen A1 bis A7 integrierte Operationsverstärker herangezogen wurden, bedarf der Komparator A8 wegen der geforderten Empfindlichkeit eines anderen Bausteines, z.B. des handelsüblichen Bausteines LF 356. Das Ausgangssignal von A8 ist entweder 0 Volt oder 12 Volt.

Jedes Atmen führt damit am Ausgang von A8 zu einem Spannungsimpuls, welcher Impuls auf dem Anzeigegerät angezeigt, einer grünen Leuchtiode zugeführt und an ein folgendes Zeitglied weitergeleitet wird. Dieses Zeitglied besteht aus einem Kondensator, z.B. einer Kapazität von 10 µF zum Negativpol und einem Drehpotentiometer. Dem Potentiometer ist ein Widerstand von z.B. 100 k Ohm vorgeschaltet. Das Drehpotentiometer ermöglicht eine Zeitintervalleinstellung von 0 bis 35 Sekunden. Ein weiterer Kondensator, der am Pluspol hängt, hat folgende Funktion:

Wenn das Gerät längere Zeit nicht eingeschaltet ist, liegt ohne diesen zweiten Kondensator das Spannungsniveau vom ersten Kondensator (am Minuspol) immer unterhalb der eingestellten Referenzspannung am Eingang von A10. Das würde dazu führen, daß das Alarm auslösende Relais Alarm gibt, da das Relais selbsthaltend ist. Der zweite Kondensator (ein Pluspol) hebt nun das Spannungsniveau am Eingang 2 über die Referenzspannung am Eingang 3 von A10. Dadurch wird der Effekt, daß das Gerät direkt nach dem Einschalten Alarm gibt, unterdrückt. Die eingestellte Referenzspannung sollte im Bereich zwischen 0,5 und 1 Volt liegen. Jeder Spannungsimpuls von A8 führt somit zur Aufladung des am Minuspol angeschlossenen Kondensators. Dieser Kondensator entlädt sich über den 100 k Ohm-Widerstand und den Drehpotentiometer in der Frontplatte. Bleiben Spannungsimpulse aus, wird der Referenzwert am Eingang 3 von A10 unterschritten und A10 schaltet durch zum Relais. Das Relais ist selbsthaltend. Es verursacht das Einschalten der roten Leuchtdiode (Alarm) sowie des Alarmtones. Wahlweise kann bei intelligenteren Versionen der Baustein A8 und A10 durch ein Mikrorechnersystem ersetzt sein.

Die symmetrische Spannungsversorgung, die für den Betrieb des Operationsverstärkers von A1 bis A7 unerläßlich ist, wird durch den Baustein A11 erzeugt. Das Gerät wird gespeist durch ein stabilisiertes Netzteil mit der Ausgangsspannung 12 Volt. Der Primärkreis des Netzteiles wird abgesichert durch eine 10 mA-Sicherung. Die Ausgangsspannung des Netzteiles liegt bei 209 und 210 an. Diese Kontakte können auch nach außen geführt werden zur externen Versorgung mit einem externen Netzteil oder einer externen 12 Volt-Batterie. Dies kann z.B. notwendig sein, wenn die Vorrichtung im Auto betrieben wird. Der Niedervoltbereich wird nochmals durch eine 100 mA-Sicherung abgesichert.

Diese Sicherung ist mit zwei 20 Volt Zenerdioden a 2,5 Watt gekoppelt. Diese haben folgende Funktion:

Würde der Netztrafo einen Schluß von der Primär- auf die Sekundärentwicklung bekommen, würde, für den Fall, daß die Stromaufnahme des Netzgerät im Primärbereich unter 10 mA bleibt, eine Spannung von 220 Volt im Maximum an 209 und 210 anliegen. Da diese Spannung größer 20 Volt ist, würden die Zenerdioden sofort durchschalten und damit die Sicherung von 100 mA durchbrennen lassen. Jede der 20 Volt Zenerdioden ist dazu in der Lage.

Somit verfügt das System zum Schutz des Kindes gegen Überspannungen über folgende Einrichtungen im Gerät:

1. eine 10 mA-Sicherung vor dem Netzgerät,

2. einen doppelpoligen Ein- und Ausschalter a11 und a12,

3. eine galvanische Trennung 220 V und 12 V durch Primärund Sekundärwicklung,

4. eine 100 mA-Sicherung vor dem Zugang zum Niedervoltteil,

5. zwei Zenerdioden, die unabhängig voneinander arbeiten, zum Auslösen de Sicherung im Niedervoltbereich,

6. Arbeiten des Sensoreingangsteils im µV-Bereich.

Zusätzlich in der Matratze:

1. PVC-Isolierfolie um die gesamte Matte,

2. Aluminiumfolie geerdet um den gesamten Kondensator.

Da die Forderung besteht, daß bei Notbetrieb/Batteriebetrieb alle unnötigen Anzeigen ausgeschaltet werden sollten, und zwar zur Energieersparnis und somit zur Verlängerung der möglichen Notbetriebsszeit, werden die Anzeigen für Netzspannung (15) und Atemfunktion (14) lediglich in den Stromkreis des Netzgerätes gebracht, d.h., bei Ausfall des Netzgerätes kann der Strom aus diesen beiden Anzeigen nicht mehr an Masse abfließen, da eine Diode den Stromkreis sperrt. Die Minuspole des Drehspulinstrumentes (11) und des Batterieblocks (12) sowie der roten Leuchtdiode (Alarm) (15) sind davon nicht betroffen. Das Netzgerät mit einer Ausgangsspannung von 12 Volt-Gleichspannung speist gleichzeitig die Notbatterie, die an (8)=Pluspol und an Punkt (12)=Minuspol angeschlossen wird. Es ist ein zusätzlicher Widerstand vorgesehen, der die Funktion hat, die Strombegrenzung beim Dauerladen der Batterie zu bewirken. Die Normalspannung der Batterie beträgt etwa 10,8 Volt, so daß immer ein ausreichendes Potentialgefälle besteht, z.B. aus 9 Zellen a 1,2 Volt wiederaufladbarer Zellen. Durch den auf 100 Ohm ausgelegten Widerstand wird selbst bei total leerer Batterie der Ladestrom auf 120 mA begrenzt. Im Normalbetrieb wird der Ladestrom der Batterie ca. 20 mA betragen. Die angenommene Potentialdifferenz beträgt dabei 2 volt. Dem 100 Ohm-Widerstand ist eine Diode nachgeschaltet, die verhindert, daß die Batterie sich in nicht eingeschaltetem Zustand über die Sekundärwicklung des Transformators oder über die Leckströme der Zenerdioden entleeren kann.

Die Gerätekonzeption sieht vor, daß im Gehäuse ein Geräuschübertragungsmelder integriert ist. Das Alarmsignal kann für diesen Fall am Punkt 207 der Schaltung abgegriffen werden. Es ist sinnvoll, den Geräuschübertragungsmelder und das Gerät über ein zentrales stabilisiertes Netzgerät zu versorgen. Das Netzgerät würde dann am Punkt 209, 210 die Schaltung speisen. Der Geräuschübertragungsmelder sollte folgende Funktionen erfüllen:

Übertragen des Alarms (immer);

Übertragen der Geräusch im Kinderzimmer (Selektion sollte möglich sein durch Festlegen der Ansprechschwelle).

Durch die Integration des Senders in das Gehäuse wird der Gefahr vorgebeugt, daß Sender und Empfänger mit fast gleichem Design vertauscht werden. Ggf. mag es auch sinnvoll sein, daß der Geräuschmelder allein betrieben werden könnten, wenn das Kind nämlich aus dem Risikoalter herausgewachsen ist und keiner Kontrolle der Atmung mehr bedarf.

Die Erfindung ist nicht auf die Ausführungsform beschränkt, sondern im Rahmen der Offenbarung vielfach variabel.

Alle neuen, in der Beschreibung und/oder Zeichnung offenbarten Einzel- und Kombinationsmerkmal werden als erfindungswesentlich angesehen.

## Patentansprüche

1. Vorrichtung zur Messung der Lebensfunktionen eines Menschen, insbesondere eines Säuglings, mit einer Sensormatte bestehend aus einer beidseitig elektrisch leitenden, mittleren Kondensatorplatte (1), oben und unten umgeben von je einem abstandshaltenden Medium (2, 3), an das obere Medium (2) anschließend eine beidseitig leitende, obere Kondensatorplatte (3a) und an das untere Medium (3) anschließend eine beidseitig leitende untere Kondensatorplatte (3b), und weiter umgeben von einer abschirmenden Schutzfolie (4), die nach außen hin elektrisch leitend und nach innen elektrisch isolierend ist, und mit einer Meß- und Anzeigevorrichtung, bestehend aus einer Verstärkereinheit und einer Auswerte- und einer Alarmeinheit, wobei die mittlere Kondensatorplatte (1) und die Schutzfolie (4) an die gemeinsame Masse (E) angelegt werden und die obere Kondensatorplatte (3a) und die untere Kondensatorplatte (3b) über Vorverstärker (A1, A2) mit den Eingängen eines Differenzverstärkers (A3) verbunden sind, wobei das Ausgangssignal des Differenzverstärkers über einen Tiefpaß und einen passiven Hochpaß einer Verstärkerstufe (A5) zugeführt ist, deren Ausgangssignal über einen weiteren Tiefpaß (A6) und einen weiteren passiven Hochpaß einer weiteren Verstärkerstufe (A7) zugeführt ist, wobei das Ausgangssignal einer Rechnerschnittstelle zuführbar ist und alternativ oder kumulativ über einen Komparator (A8) mit einstellbaren Referenzwerten auswertbar ist, dessen Ausgangssignal mindestens ein Anzeigegerät und gegebenenfalls eine oder mehrere Leuchtdioden steuert, wobei das Ausgangssignal zudem ein Zeitglied steuert, welches vorzugsweise aus einem Kondensator mit ca. 100 µF Kapazität und einem regelbaren 100 k Ohm Widerstand besteht, wobei ferner das Ausgangssignal unmittelbar und mittelbar über das Zeitglied einem weiteren Komparator zugeführt wird, der ein Relais schaltet, sofern der eingestellte Referenzwert des Komparators unterschritten wird, wobei insbesondere ferner das Relais selbsthaltend ausgebildet ist und eine Signaleinrichtung steuert.

2. Vorrichtung nach Anspruch 1 dadurch gekennzeichnet, daß die Sensormatte auch Temperatur- und Feuchtigkeitssensoren aufweist.

3. Vorrichtung nach Anspruch 1 und 2 dadurch gekennzeichnet, daß die Sensormatte zur Lageerkennung des Menschen in verschiedene Felder aufgeteilt ist.

4. Vorrichtung nach Anspruch 1 dadurch gekennzeichnet, daß zur genauen Differenzierung des Pulsschlages von der Atmungstätigkeit ein insbesondere autonomer Sender am Körper des Kindes oder dgl. befestigt ist, mittels dessen der Pulsschlag drahtlos auf die Sensormatte übertragen wird, daß die Sensormatte eine entsprechende Empfangseinheit für dieses Signal aufweist und daß die Signale gemeinsam mit den Atmungssignalen in die Auswerteeinheit überführt und dort entsprechend dem Frequenzunterschied und Bildmusteranalyse in Puls- und Atmungssignale aufgetrennt und verarbeitet werden.

5. Vorrichtung nach Anspruch 1—4 dadurch gekennzeichnet, daß sie eine zentrale drahtlos gesteuerte Anzeigeneinheit aufweist, diese weist mehrere Anzeigeneinheiten auf, die den jeweiligen Lebenszustand einer Vielzahl von Menschen wiedergeben und Einzelalarme auslösen.

## Revendications

1. Dispositif de mesure des fonctions organiques d'un être humain, en particulier d'un nourrisson, comportant une natte de détection constituée d'une plaque de condensateur médiane (1) conductrice de l'électricité des deux côtés, entourée au-dessus et au-dessous par un élément séparateur (2, 3), une plaque de condensateur supérieure (3a) conductrice de l'électricité de chaque côté, reliée à l'élément supérieur (2), et une plaque de condensateur inférieure (3b) conductrice de l'électricité de chaque côté, reliée à l'élément inférieur (3), l'ensemble étant en outre entouré d'une feuille de protection (4) qui est conductrice de l'électricité vers l'extérieur et isolante vers l'intérieur, et un dispositif de mesure et d'affichage constitué d'une unité d'amplification ainsi que d'une unité d'évaluation et d'une unité d'alarme, la plaque de condensateur médiane (1) et la feuille de protection (4) étant reliées à la masse commune (E), et la plaque de condensateur supérieure (3a) et la plaque de condensateur inférieure (3b) étant reliées via un pré-amplificateur (A1, A2) aux bornes d'entrée d'un amplificateur différentiel (A3), et sorte que le signal de sortie de l'amplificateur différentiel soit acheminé via un filtre passe-bas et un filtre passe-haut passif à un étage d'amplification (A5) dont le signal de sortie est envoyé via un autre filtre passe-bas (A6) et un autre filtre passe-haut passif à un autre étage d'amplification (A7), le signal de sortie pouvant être envoyé à un interface d'ordinateur et pouvant être évalué de manière alternée ou cumulative via un comparateur (A8) à valeurs de référence réglables, dont le signal de sortie commande au moins un appareil d'affichage et éventuellement une ou plusieurs diodes photoluminescentes, le signal de sortie commandant en outre une minuterie qui est constituée de préférence d'un condensateur d'une capacité d'environ 100 µF et d'une résistance de 100 kOhm réglable, ce signal de sortie pouvant par ailleurs être envoyé indirectement et directement via la minuterie à un autre comparateur qui commute un relais dès l'instant où la valeur de référence réglée du comparateur est dépassée, le relais étant par alleurs conformé de manière automono et commantant un dispositif de signalisation.

2. Dispositif selon la revendication 1, caractérisé en ce que la natte de détection présente également des détecteurs de température et d'humidité.

3. Dispositif selon les revendications 1 et 2, caractérisé en ce que la natte de détection est répartie en plusieurs champs pour apprécier les conditions générales de l'individu.

4. Dispositif selon la revendication 1, caractérisé en ce que, pour différencier exactement l'impulsion du pouls de la fonction respiratoire, un émetteur autonome spécial est fixé au corps de l'enfant ou du patient, grâce auquel l'impulsion du pouls est transmise sans fil à la natte de détection, la natte de détection présente une unité de réception correspondante pour ce signal, et les signaux conjoints avec les signaux respiratoires sont transmis à l'unité d'évaluation et y sont séparés et traités en fonction de la différence de fréquence et de l'analyse de structure pour les différencier en signaux de pouls et signaux de respiration.

5. Dispositif selon les revendications 1 à 4, caractérisé en ce qu'il présente une unité d'affichage centrale commandée sans fil, qui comporte elle-même plusieurs éléments d'affichage qui reproduisent l'état organique correspondant d'un certain nombre individus, et déclenche des alarmes individuelles.

## Claims

1. A device for measuring the vital function of a human being, especially of a baby, comprising a sensor pad consisting of a middle capacitor plate (1) which is electrically conductive on both sides and is surrounded above and beneath by a spacing medium (2, 3), an upper capacitor plate (3a), which is conductive on both sides, connecting with the upper medium (2), and a lower capacitor plate (3b), which is conductive on both sides, connecting with the lower medium (3), which in addition are surrounded by a covering protective sheet (4) which is electrically conductive on the outside and electrically insulating on the inside, and comprising a measuring and displaying device consisting of an amplification unit and an evaluation unit and an alarm unit, wherein the middle capacitor plate (1) and the protective sheet (4) are connected to a common earth (E) and the upper capacitor plate (3a) and the lower capacitor plate (3b) are connected through preamplifiers (A1, A2) to the inputs of a differential amplifier (A3) and the output signal of the differential amplifier is supplied through a low-pass filter and a passive high-pass filter to an amplification stage (A5), the output signal of which is supplied through another low-pass filter (A6) and another passive high-pass filter to another amplification stage (A7), where the output signal can be supplied to a computer interface and alternatively or cumulatively can be evaluated by means of a comparator (A8) having adjustable reference values, the output signal of which controls at least one display device and optionally one or more light-emitting diodes, where the output signal also controls a time function element, which preferably consists of a ca. 100 μF capacitor and a variable 100 k ohm resistor, and where moreover the output signal is supplied directly and indirectly through the time function element to another comparator which switches a relay when the preset reference value of the comparators is not reached, where moreover the relay is, in particular, constructed to a self-locking relay and controls a signalling device.

2. A device according to Claim 1, characterized in that the sensor pad also comprises temperature and moisture sensors.

3. A device according to Claims 1 and 2, characterized in that the sensor pad is divided into different sections in order to determine the condition of the human being.

4. A device according to Claim 1, characterized in that, for accurate differentiation of the pulse beat from the respiratory activity, a special autonomous transmitter is fastened to the body of the child or other person, by means of which the pulse beat is radio transmitted to the sensor pad, the sensor pad comprises a corresponding receiver unit for these signals and these signals together with the respiration signals are passed to the evaluation unit and, according to the difference in frequency and the pattern analysis, are separated into pulse and respiration signals and processed there.

5. A device according to Claims 1—4 characterized in that the device comprises a central radio-controlled display unit which comprises a plurality of display elements which show the respective states of the vital functions of several persons and trigger individual alarms.

Atmung

$\frac{T}{2}$

$T_{atmung}$

*Fig. 1*

Puls

$\frac{T_1}{2}$ $\frac{T_2}{2}$ $\frac{T_3}{2}$ $\frac{T_4}{2}$

$\frac{T_4}{2}$

$T_{puls}$

*Fig. 2*

Temperatur

*Fig. 3*

Muskelkontraktion

Amplitude

*Fig. 4*

1

Fig. 5

Fig. 6

Fig. 7

Fig. 8

EP 0 205 931 B1